(19) Europäisches Patentamt
European Patent Office
Office européen des brevets

(11) **EP 2 879 648 B1**

(12) **EUROPEAN PATENT SPECIFICATION**

(45) Date of publication and mention
of the grant of the patent:
**31.03.2021   Bulletin 2021/13**

(21) Application number: **13742215.0**

(22) Date of filing: **25.07.2013**

(51) Int Cl.:
*A61K 8/25* *(2006.01)*          *A61K 8/26* *(2006.01)*
*A61K 8/31* *(2006.01)*          *A61K 8/73* *(2006.01)*
*A61K 8/81* *(2006.01)*          *A61Q 19/10* *(2006.01)*
*A61K 8/92* *(2006.01)*          *A61K 8/04* *(2006.01)*
*A61K 8/9789* *(2017.01)*

(86) International application number:
**PCT/EP2013/065766**

(87) International publication number:
**WO 2014/019944 (06.02.2014 Gazette 2014/06)**

(54) **FORMULATION FOR GRITTY FOAM DISPENSER**

FORMULIERUNG FÜR EINEN SPENDER FÜR GROBKÖRNIGEN SCHAUM

FORMULATION POUR DISTRIBUTEUR DE MOUSSE GRANULEUSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.08.2012   US 201261679377 P
14.03.2013   US 201313804209**

(43) Date of publication of application:
**10.06.2015   Bulletin 2015/24**

(73) Proprietor: **Deb IP Limited
Denby, Derbyshire DE5 8JZ (GB)**

(72) Inventors:
• **GRIMADELL, Louise
Derby
Derbyshire DE22 2NU (GB)**
• **CREAGHAN, David Michael Ross
Derby
Derbyshire DE22 1DG (GB)**

• **HINES, John D.
Audlem
Cheshire CW3 0BA (GB)**
• **GRASCHA, Pierre Bruno
F-51350 Cormontreuil (FR)**

(74) Representative: **Elsy, David et al
Withers & Rogers LLP
4 More London Riverside
London SE1 2AU (GB)**

(56) References cited:
**WO-A1-00/37597          WO-A1-91/14420
WO-A1-03/095600          US-A- 5 661 119
US-A1- 2005 137 102          US-A1- 2011 251 277**

Remarks:
The file contains technical information submitted after the application was filed and not included in this specification

## Description

## FIELD

The present disclosure relates to formulations for a gritty foam dispenser.

## BACKGROUND

Currently, there are three (3) broad categories of hand cleansers that form the away from home skincare market. They are: lotion soaps, lotion soaps with suspended mechanical scrubbers and foam soaps. Lotion soaps are generally dispensed from soap dispenser pumps or pump top bottles. Lotion soaps with mechanical scrubbers are also dispensed through similar devices. Foaming soaps are dispensed from special pumps that mix air and liquid together through a porous media to produce foam.

Hand cleansers have been around for many years. The combination of ingredients in these types of soaps can be specified to achieve a broad range of cleaning characteristics. The viscosity of this form of soap is in the range of 5,000 to 30,000 cPoise and is generally tailored to be easily manipulated in the user's hand or pumped through a pump.

With the addition of mechanical scrubbers, the cleaning performance of lotion soaps can be enhanced. Lotion soaps with suspended mechanical scrubbers tend to have a viscosity range at the low end that is higher than that of basic lotion soaps. In order to provide the structure to suspend mechanical scrubbers of an appropriate size to provide cleaning benefits, these formulations must also have a critical strain force (or yield value) superior or equal to 30 dynes / $cm^2$ associated or not to non-Newtonian rheological behaviour, and more particularly plastic or thixotropic properties (pseudoplastic fluids being excluded from this requirement). The rheology of this form of soap must be viscoplastic (Casson, or Bingham, or dilatant, or thixotropic, or rheopectic fluids) with a critical strain force which is preferably greater than or equal to 30 dynes/$cm^2$. The critical strain force is defined as that stress which must be applied before flow will start and, although related to viscosity, it is more dependent on the characteristics of the rheological additive used. The critical strain force has traditionally been determined by measuring the viscosity of the material at two speeds using a Brookfield Viscometer. As the speed of rotation of the spindle is increased materials that undergo shear thinning give a lower viscosity measurement.

The soap's critical strain force or the required yield value may be calculated as follows:

$$\text{Yield Value (in dynes/cm}^2\text{)} = (V_{0.5rpm} - V_{1rpm}) / 100$$

$$\text{Required Yield Value (in dynes/cm}^2\text{)} = [4/3\ R(D-Do)g]$$

Where:

*R = particle radius (cm)*
*D = particle density (g/cm$^3$) Do = medium density (g/cm$^3$)*
*g = acceleration due to gravity = 980 cm/s$^2$*

Foaming hand cleansers offer many benefits over lotion hand cleansers. Foam products are quicker and easier to use since they are delivered into the user's hand in a lathered format. Foam hand cleansers spread more effectively than lotion hand cleansers, providing better cleaning performance with less product. Since foaming hand cleansers are already lathered, there is less water needed in the cleaning process and the user can clean their hands in less time. The viscosity range of this form of hand cleanser is less than 100 cPoise and is tailored to be easily mixed with air through a porous media to produce foam from a pump.

The characteristics of foam soaps are such that they are not capable of suspending mechanical scrubbers of sufficient size to provide cleaning benefits. Current foam pump technologies mix air and liquid together and pass them through a porous media to make the foam. The porous media is generally very fine and would sieve out mechanical scrubbers.

Recently, a new type of pump capable of dispensing hand cleansers with mechanical scrubber in a foam format through a non-aerosol dispensing system has been developed (see United States Patent Nos. 8,002,151 and 8,281,958). This has allowed for the creation of a new hand cleanser category. This category is foaming hand cleansers with mechanical scrubbers. This new category will allow the leveraging of all the benefits associated with foaming hand cleansers with the enhanced cleaning performance provided by mechanical scrubbers. Nevertheless, the requirement remains for a suitable hand cleanser.

Thus, it would be very beneficial if a hand cleanser could be formulated that can suspend particulate mechanical scrubbers with particles sized from about 100 microns to about 800 microns that decreases in viscosity when exposed to high shear rates and is capable of being foamed through a non-aerosol, or unpressurized pump dispenser.

If the formulation is too thin, the mechanical scrubbers will fall out of suspension. If the product is too thick, the amount of force required to foam the formulation become too high resulting in excessive operating force for the dispenser user and poor quality foam.

Prior art does not capture the requirement of the formulation to generate foam when mixed and dispensed with air from a non-aerosol dispenser. The assumption from the prior art is since it is soap, it will naturally foam. While this may be true if mechanical manipulation is done (rubbing in the hand), if the formulation does not have the desired characteristics, it will not create an acceptable foam through a non aerosol pump.

## SUMMARY

Provided herein are formulations for a gritty foam dispenser. An embodiment of a foamable gritty composition comprises constituents including a solvent present in a range from about 0.5% w/w to about 30.0% w/w, in which the solvent includes any one or combination of D'limonene and sunflower oil methyl ester. The formulations include a particulate scrubbing agent present in a range from about 1 .0% w/w to about 8% w/w. Included is a surfactant present in a range from about 0.5% w/w to about 30.0% w/w, skin conditioner present in a range from 0.01% w/w to about 5.00% w/w, a non-Newtonian thickening agent present in a range from about 1,5% w/w to 1,7% w/w, and water, wherein the non-Newtonian thickening agent is selected from: acrylates/C10-30 alkyl acrylate crosspolymer, carbomers, xanthan gum, guar gum, quaternised guar gum, alginate, bentonite, fumed silica or combination thereof. The non-Newtonian thickening agent is selected to give the foamable gritty formulation a critical strain force greater than or equal to about 30 dynes/cm$^2$, and a viscosity in a range from about 500 cPoise to about 4000 cPoise such that the foamable gritty composition is dispensible as a foam from a non-aerosol foam dispenser.

The present disclosure also provides a method of producing and dispensing a gritty foam, the method comprising; dispensing a foamable gritty composition from an unpressurized container having a dispenser pump configured to mix air with the foamable gritty composition under low pressure conditions during dispensing to form a gritty foam, the foamable gritty composition comprising

a) constituents including

a solvent present in a range from about 0.5% w/w to about 30.0% w/w, said solvent including any one or combination of D'limonene and sunflower oil methyl ester;
a particulate scrubbing agent present in a range from about 1.0% w/w to about 8% w/w;
a surfactant present in a range from about 0.5% w/w to about 30.0% w/w;
skin conditioner present in a range from 0.01% w/w to about 5.00% w/w; and
a non-Newtonian thickening agent present in a range from about 0.05% w/w to about 10% 1,5% w/w to 1,7 w/w; water; and

b) wherein the non-Newtonian thickening agent is selected from: acrylates/C10-30 alkyl acrylate crosspolymer, carbomers, xanthan gum, guar gum, quaternised guar gum, alginate, bentonite, fumed silica or combination thereof. The non-Newtonian thickening agent is selected to give the foamable gritty composition a critical strain force greater than or equal to about 30 dynes/cm$^2$, and a viscosity in a range from about 500 cPoise to about 4000 cPoise such that said foamable gritty composition is dispensible as a foam from a an unpressurized foam dispenser.

A further understanding of the functional and advantageous aspects of the disclosure can be realized by reference to the following detailed description and drawings.

## BRIEF DESCRIPTION OF THE TABLES

Embodiments will now be described, by way of example only, with reference to the drawings, in which:

Table 1 shows a broad class of constituents that can be used to produce formulations for a gritty foam dispenser in accordance with the present invention;
Table 2 shows an exemplary formulation.

## DETAILED DESCRIPTION OF THE INVENTION

Various embodiments and aspects of the disclosure will be described with reference to details discussed below. The following description and drawings are illustrative of the disclosure and are not to be construed as limiting the disclosure. Numerous specific details are described to provide a thorough understanding of various embodiments of the present disclosure. However, in certain instances, well-known or conventional details are not described in order to provide a concise discussion of embodiments of the present disclosure.

**Definitions:**

As used herein, the terms, **"comprises"** and **"comprising"** are to be construed as being inclusive and open ended, and not exclusive. Specifically, when used in this specification including claims, the terms, "comprises" and "comprising" and variations thereof mean the specified features, steps or components are included. These terms are not to be interpreted to exclude the presence of other features, steps or components.

As used herein, the term **"exemplary"** or **"example"** means "serving as an example, instance, or illustration," and should not be construed as preferred or advantageous over other configurations disclosed herein.

As used herein, the terms **"about"** and **"approximately",** when used in conjunction with ranges of dimensions of particles, compositions of mixtures or other physical properties or characteristics, are meant to cover slight variations that may exist in the upper and lower limits of the ranges of dimensions so as to not exclude embodiments where on average most of the dimensions are satisfied but where statistically dimensions may exist outside this region. It is not the intention to exclude embodiments such as these from the present disclosure.

As used herein, the phrase **"non-Newtonian thickener"** or "non-Newtonian thickening agent" means a category of fluids whose viscosity (resistance to deformation or shear forces) is influenced by the shear stress and/or by the time this force is applied.

As used herein, the phrase "**critical strain force**" (also referred to as "**yield value**") refers to a term used in rheology in which the 'critical strain force' is the minimum force that must be applied to a material to induce viscous flow. The critical strain force is a measurable quantity (expressed in dynes/cm$^2$). More particularly, both phrases are referring to the same phenomenon which is that up to a certain force exerted (the critical strain force), a visco-elastic material will exhibit "solid like" or "elastic" properties, i.e. it will not have a net flow. Above the critical strain force (or above the "yield value") the same material will exhibit viscous properties, i.e. it will flow.

As an example, for beads (particulate scrubber particles in the present context) suspended in a fluid, as long as the strain force imparted by the suspended beads is less than the critical strain force (or less than the yield value), the beads will stay forever in suspension. If they exert a force higher than the critical strain (or yield value), then they will migrate over time, either floating to the top of the fluid or settling to the bottom.

As used herein, the phrase "non-aerosol dispenser" or "unpressurized dispenser" means a dispenser that does not have a stored propellant as the source of gas to create the foam structure within the hand cleanser.

"Foam" as used herein means a liquid and a gas mixed to form a mass of small bubbles that has a structure that lasts for a variable length of time.

The phrase "low pressure" in the context of producing a foam as used herein means a pressure of around an atmosphere or less such as when dispensing the foam from an unpressurized container. Typically when foams are dispensed from aerosol containers the foam is considered to be being dispensed under high "pressure" conditions.

The present disclosure provides a skin cleanser, which is a foamable gritty composition that can suspend mechanical scrubber particles having a size in the range from about 100 to about 800 microns that decreases in viscosity when exposed to high shear rates and is capable of being foamed through a non-aerosol (unpressurized) pump.

If the formulation is too thin (viscosity too low) and has a Newtonian rheological behaviour, the mechanical scrubbers will fall out of suspension. If the product is too thick (too viscous), the amount of force required to foam the formulation becomes too high resulting in excessive operating force for the dispenser user and a poor quality foam results.

While it is assumed in the field that if surfactants are present, it will naturally foam. However while this is a necessary condition, the inventors have found that it is not a sufficient condition to obtain a foam when particulate scrubbing agents are present. First, the fluid must be capable of becoming aerated and second it must be capable of stabilising the entrained air bubbles divided by fluid films. The presence of surfactants in a suitable concentration broadly satisfies the second of these requirements by providing a means of stabilising fluid films by setting up a surface tension gradient that acts to oppose draining forces and hence maintains the thickness of the films, avoiding collapse.

However, the presence of surfactants alone does not meet the first requirement, which requires that the fluid is sufficiently low in viscosity to allow for mixing with air under the conditions provided by the pump (i.e. without the requirement for excessive force). If the fluid is too viscous, air will not mix and hence bubbles will not form (and then cannot be stabilised as foam). This issue is central to providing a fluid of sufficiently low viscosity to allow for efficient mixing and hence foam formation, and also to contribute to suspending particles in the fluid when at rest.

**Constituents**

**Solvents**

In addition, the solvent can include aqueous (water) and non-aqueous components. The non-aqueous solvent is one or both of D-limonene and sunflower oil methyl ester. Other optional solvents that can be added include any one or combination of glycol ethers, esters, alcohols, terpenes other than D'Limonene, aromatic-free white spirit. A total amount of non-aqueous solvent present in the composition is in the range from about 0.5% w/w to about 30.0% w/w. The water is present in an amount to balance the total composition to 100% w/w weight.

**Scrubbing Agent**

The particulate scrubbing agent is any one or combination of a vegetable based scrubbing agent, a synthetic based scrubbing agent and a mineral based scrubbing agent, and the particles have a size in a range from about 100 microns to about 800 microns, or in a range from about, or in a range from about 200 to about 700, or in a range from about 300 to about 500 microns. The particles may have any size in this range, or be in any narrow range than 100 to 800 microns, or there may be a mixture of any size of particles in this broad range present in the composition. The vegetable based scrubbing agent may be any one or combination of cornmeal, olive stone, walnut shells, ground fruit stones, ground corn meal, ground fruit shells. The synthetic based scrubbing agent may be any one or combination of polyethylene and polypropylene. The mineral based scrubbing agent may be any one or combination of ground shellfish, pumice, and silica. The particulate scrubbing agent present is present a range from about 1.0% w/w to about 8% w/w.

**Surfactants**

The surfactant may include any one or combination of i) an anionic surfactant present in a range from about 1% w/w to about 20% w/w if present alone; ii) an amphoteric surfactant present in a range from about 0.5% w/w to about 5.0% w/w if present alone; iii) a non-ionic surfactant present in a range from about 0.5% w/w to about 20% w/w if present alone. If the surfactant is any combination of two or more of i), ii) or iii), the total amount of surfactant is also in the range from about 0.5% w/w to about 30.0% w/w.

**i) Amphoteric Surfactants**

The amphoteric surfactant may be any one or combination of betaines, acyl ethylene diamines, amino-acids derivates, imidazolines. Alternatively, the amphoteric surfactant may be any one or combination of acylamphoacetate, acylamphodiacetate, acylamphodipropionate, sodium cocoglycinate, sodium alkyliminodipropionate, cocamidopropyl betaine, sodium cocoamphoacetate.
The betaine may be any one or combination of coco betaine and cocamidopropyl betaine.

**ii) Non-ionic Surfactants**

The non-ionic surfactant may be any one or combination of of glucosides, ethoxylated fatty alcohols, ethoxylated fatty acids, saccharose esters, sorbitan esters, alkanolamides, glycerol alkyl esters, polyoxyethylene glycol alkylphenol ethers,

**iii) Anionic Surfactants**

The anionic surfactant may be any one or combination of lauryl sulphates, lauryl ether sulphates, sulphosuccinates, carboxylates (i.e. sodium oleate), carboxylic acid esters (i.e. sodium dilaureth citrate), alkyl sulfate (i.e. sodium lauryl ether sulfate, ammonium alkyl sulfate, alkyl and alkyl-aryl sulfonates (i.e. sodium dodecyl benzene sulfonate), sulfosuccinates (i.e. disodium lauryl ether sulfosuccinate), isethionates (i.e. sodium cocoyl isethionate, ammonium cocoyl isethionate), taurates (i.e. sodium methyl cocoyl taurate, sodium methyl oleoyl taurate), acyl glutamates (i.e. sodium lauroyl glutamate, sodium cocoyl glutamate, disodium cocoyl glutamate), sarcosinate (i.e. cocoyl sarcosinate), alkylpolyglucosides (i.e. decyl glucoside, sodium lauryl glucose carboxylate, caprylyl/capryl glucoside).

**Skin Conditioner**

The skin conditioner is any one or combination of a polyol, an anionic surfactant (examples: methyl gluceth-20, sodium laureth carboxylate, xylitylglucoside, sodium cocoyl glutamate, sodium cocoyl glycinate) a non-ionic surfactant (examples: PEG castor oil, PEG dimethicone, Glycereth cocoate), a cationic surfactant (examples: cocamidopropyl betainamide mea chloride, cocamidopropyl pg-dimonium chloride phosphate), an amphoteric surfactant (examples: lauramine oxide, undecylenamidopropyl betaine, sodium cocoamphoacetate), a cationic polymer, a quaternised gum, and a polyol.

The polyol may be any one or combination of glycerine and polyglycerin-6, propylene glycol, sorbitol, mannitol, erythritol, xylitol, arabitol, ribitol, dulcitol, lactitol, and maltitol.

The skin conditioner is present in a range from 0.01 % w/w to about 5.00% w/w.

**Non-Newtonian Thickening Agents**

The non-Newtonian thickening agent is selected to give the foamable gritty composition a critical strain force greater than or equal to about 30 dynes / $cm^2$, and a viscosity in a range from about 500 cPoise to about 4000 cPoise, and preferably about 1000 cPoise to about 3000 cPoise, and most preferably about 2000 cPoise to about 2500 cPoise such that the foamable gritty composition is dispensible as a foam from a non-aerosol foam dispenser.

The non-Newtonian thickening agent is any one or combination of synthetic polymers and natural thickeners. The synthetic polymer is any one or combination of acrylates/C10-30 alkyl acrylate crosspolymer and carbomers.

The natural thickener is any one or combination of xanthan gum, guar gum, quaternised guar gum, alginate, bentonite and fumed silica.

The non-Newtonian thickening agent is present in a range from 1,5% w/w to 1,7% w/w.

**Antioxidants**

Antioxidants may be included in the formulations which are included to limit the risk of oxidation of d-limonene (becomes a sensitizer when oxidised). The antioxidants may include, but are not limited to: butylhydroxytoluene (BHT), butylhydroxyanizole, 1,2-dihydroxybenzene, p-coumarine acid, caffeic acid, sodium sulfite, sodium metasulfite, ferrulic acid, tyrosol, quercetin, chlorogenic acid, oleuropein hydroxytyrosol, ascorbic acid, phenolic acid, propyl gallate, $\alpha$-tocopherol, $\beta$-tocopherol, $\gamma$-tocopherol, $\delta$-tocopherol, tetradibutyl-pentaerithrityl hydroxyhydrocinnamate. The antioxidants may be present in an amount ranging from about 0.01 to 1.0% w/w.

**Non-aerosol, Unpressurized Pump**

A useful non-aerosol, unpressurized pump that may be used is disclosed in United States Patent Nos. 8,002,151 and 8,281,958, which are incorporated herein by reference in their entirety. The unpressurized container used for dispensing the present foamable gritty composition will generate pressure when the gritty composition and air are introduced into a mixing chamber by mechanical actuation of a dispensing feature(s), and this mechanical actuation can be initiated by a user actuating the dispensing feature(s) or by sensor activated dispensing feature(s) when the presence of a user is detected.

Using such an unpressurized pump, the present disclosure provides a method of making and dispensing a gritty foam. The method involves introducing a predetermined quantity of air under pressure to a first side of a microporous air sparging element located upstream from an outlet while introducing, concurrently with the introduction of the air, a predetermined amount of the foamable gritty composition under pressure to a foamable gritty composition inlet of a mixing chamber located on a second side of the microporous air sparging element and located upstream from the outlet, such that a ratio of air to foamable gritty composition has a greater proportion of air to foamable gritty composition. The method involves forcing the air through the microporous sparging element into the mixing chamber to form a plurality of bubbles and mixing the plurality of bubbles and the foamable gritty composition in the mixing chamber thereby creating a gritty foam and dispensing the gritty foam to a user in shots through the outlet from the mixing chamber.

The air may be introduced in a predetermined quantity and the foamable gritty composition may be introduced in a predetermined quantity

The specific embodiments described above have been shown by way of example, and it should be understood that these embodiments may be susceptible to various modifications and alternative forms. It should be further understood that the claims are not intended to be limited to the particular forms disclosed, but rather to cover all modifications, equivalents, and alternatives falling within the spirit and scope of this disclosure.

**TABLE 1**

| Formulation to contain | In the range (%w/w) | | | |
|---|---|---|---|---|
| | **Typically** | **Preferably** | **More preferably** | **Specific examples** |
| Thickener/ suspendin g agent | 0.05-10.0 | 1.0-2.0 | 1.5-1.7 | Acrylic thickeners such as acrylates copolymer, natural thickeners such as xanthan gum |
| Scrubbing agent | 1.0 - 8.0 | 4.0 - 8.0 | 6.5 - 7.5 | Vegetable based scrubbing agents such as cornmeal, olive stone, walnut shell |
| Anionic surfactants | 5.5-11.5 | 8.0 - 10.0 | 8.0 - 10.0 | Lauryl sulphates, lauryl ether sulphates, sulphosuccinates, |
| Amphoteric surfactants | 0.8 - 3.0 | 1.0 - 2.0 | 1.0 - 2.0 | Betaines such as coco betaine |
| Nonionic surfactants | 0.7 - 3.0 | 1.0 - 2.0 | 1.0 -2.0 | Coco-glucoside, decyl glucoside |
| Skin conditioner | 0.01 - 5.0 | 2.0-4.0 | 3.0 - 4.0 | Polyglycerin-6, glycerine |
| Solvent | 0.5-30.0 | 1.0-10.0 | 1.0 - 6.0 | D'Limonene, sunflower oil methyl ester |
| Water | 29.5-91.44 | 62.0-82.0 | 66.8 - 78.0 | |

**TABLE 2**

| CAS | INCI | Generic Name | Nominal Code | Nominal Active Level % | % w/w | Nominal Active Content % | Function |
|---|---|---|---|---|---|---|---|
| 7732-18-5 | Aqua | Water | 11 210 355T | 100.00 | 48.02999 | 75.23212 | Solvent/diluent |
| N/A (Polymer) | Acrylates Copolymer | Aqua SF-1 | 11 105 567T | 30.00 | 5.15000 | 1.54500 | Thickener/Suspending Agent |
| 52-51-7 | 2-Bromo-2-Nitropropane-1, 3-Diol | Euxyl K145 | 11 105 560T | 14.00 | 0.30000 | 0.04200 | Preservative |
| 2-682-20-4 | Methylisothiazolinone | | | 0.12 | | 0.00036 | Preservative |
| 29-172-55-4 | Methylchloroisothiazolinone | | | 0.37 | | 0.00111 | Preservative |
| 10377-60-3 | Magnesium Nitrate | | | <1 | | <0.003 | Preservative Stabiliser |
| 7786-30-3 | Magnesium Chloride | | | <1 | | <0.003 | Preservative Stabiliser |
| 66071-96-3 | Corn (Zea Mays) Meal | Cornmeal (irradiated) | 11 105 620T | 100.00 | 7.50000 | 7.50000 | Scrubbing Agent |
| 144538-83-0 | Tetrasodium Iminodisuccinate | Sodium Iminodisuccinate | 11 105 579T | 78.00 | 0.10000 | 0.07800 | Chelating Agent |
| 9004-82-4 | Sodium Laureth Sulfate | SLES, Preserved | 11 200 968T | 27.50 | 18.00000 | 4.95000 | Anionic Surfactant |
| 139-96-8 | TEA-Lauryl Sulfate | TEA Lauryl Sulphate | 11 105 207T | 40.00 | 8.00000 | 3.20000 | Anionic Surfactant |
| 5989-27-5 | Limonene | D'Limonene | 11 125 253T | 99.90 | 1.00000 | 0.99900 | Solvent |
| 128-37-0 | BHT | | | 0.10 | | 0.00100 | Antioxidant |
| 61789-40-0 | Cocamidopropyl Betaine | Betaine Coco Base | 11 105 204T | 30.00 | 4.50000 | 1.35000 | Amphoteric Surfactant |
| 25618-55-7 | Polyglycerin-6 | Polyglycerin-6 | 11 105 631T | 79.00 | 4.00000 | 3.16000 | Skin Conditioner |
| 56-81-5 | Glycerin | | | 1.00 | | 0.04000 | Skin Conditioner |
| 1310-73-2 | Sodium Hydroxide | Sodium Hydroxide | 11 105 107T | 47.00 | 0.12000 | 0.05640 | Neutraliser |

(continued)

| CAS | INCI | Generic Name | Nominal Code | Nominal Active Level % | % w/w | Nominal Active Content % | Function |
|---|---|---|---|---|---|---|---|
| 141464-42-8 | Coco-Glucoside | Glucopon 650 EC | 11 105 484T | 51.50 | 3.00000 | 1.54500 | Nonionic Surfactant |
| 77-92-9 | Citric Acid | Citric Acid | 11 105 114T | 100.00 | 0.30000 | 0.30000 | pH Adjuster |
| 7647-14-5 | Sodium Chloride | Sodium Chloride | 11 105 161T | 100.00 | 0.00001 | 0.00001 | Sodium chloride could possibly be needed for batch correction (Viscosity Adjuster) |
| | | Total | | | 100.00000 | 100.00000 | |

**Claims**

1. A foamable gritty composition, comprising:

   a) constituents including
   a solvent present in a range from 0.5% w/w to 30.0% w/w, said solvent including any one or combination of D'limonene and sunflower oil methyl ester;
   a particulate scrubbing agent including mechanical scrubber particles of from 100 to 800 microns in size, present in a range from 1.0% w/w to 8% w/w;
   a surfactant present in a range from 0.5% w/w to 30.0% w/w;
   skin conditioner present in a range from 0.01% w/w to 5.00% w/w; and
   a non-Newtonian thickening agent present in a range from 1.5% w/w to 1.7% w/w;
   water; and
   b) wherein the non-Newtonian thickening agent is selected from: acrylates/C10-30 alkyl acrylate crosspolymer, carbomers, xanthan gum, guar gum, quaternised guar gum, alginate, bentonite, fumed silica or combination thereof,
   such that said foamable gritty composition is dispensible as a gritty foam from a non-aerosol foam dispenser capable of dispensing hand cleansers with said mechanical scrubber particles present therein.

2. The composition according to claim 1 wherein the surfactant includes any one or combination of

   i) an anionic surfactant present in a range from 1% w/w to 20% w/w if present alone;
   ii) an amphoteric surfactant present in a range from 0.5% w/w to 5.0% w/w if present alone;
   iii) a non-ionic surfactant present in a range from 0.5% w/w to 20% w/w if present alone; and

   wherein a total amount of surfactant or any combination of surfactants present is in a range from 0.5% w/w to 30.0% w/w.

3. The composition according to claim 2 wherein the anionic surfactant is selected from the group consisting of lauryl sulphates, lauryl ether sulphates, sulphosuccinates, carboxylates (i.e. sodium oleate), carboxylic acid esters (i.e. sodium dilaureth citrate), alkyl sulfate (i.e. sodium lauryl ether sulfate, ammonium alkyl sulfate), alkyl and alkyl-aryl sulfonates (i.e. sodium dodecyl benzene sulfonate), sulfosuccinates (i.e. disodium laurylether sulfosuccinate), isethionates (i.e. sodium cocoyl isethionate, ammonium cocoyl isethionate), taurates (i.e. sodium methyl cocoyl taurate, sodium methyl oleoyl taurate), acyl glutamates (i.e. sodium lauroyl glutamate, sodium cocoyl glutamate, disodium cocoyl glutamate), sarcosinate (i.e. cocoyl sarcosinate), alkylpolyglucosides (i.e. decyl glucoside, sodium lauryl glucose carboxylate, caprylyl/capryl glucoside).

4. The composition according to claim 2 wherein the amphoteric surfactant is any one or combination of betaines, acyl ethylene diamines, amino-acids derivates, imidazolines.

5. The composition according to claim 2 wherein the amphoteric surfactant is any one or combination of acylamphoacetate, acylamphodiacetate, acylamphodipropionate, sodium cocoglycinate, sodium alkyliminodipropionate, cocamidopropyl betaine, sodium cocoamphoacetate.

6. The composition according to claim 4 wherein said betaine is selected from the group consisting of coco betaine and cocamidopropyl betaine.

7. The composition according to claim 2 wherein the non-ionic surfactant is any one or combination of of glucosides, ethoxylated fatty alcohols, ethoxylated fatty acids, saccharose esters, sorbitan esters, alkanolamides, glycerol alkyl esters and polyoxyethylene glycol alkylphenol ethers.

8. The composition according to any one of claims 1 to 7 wherein the solvent further includes any one or combination of glycol ethers, esters, alcohols, terpenes other than D'Limonene, aromatic-free white spirit, and wherein a total amount of solvent present is in said range from 0.5% w/w to 30.0% w/w.

9. The composition according to any one of claims 1 to 8 wherein the particulate scrubbing agent is any one or combination of a vegetable based scrubbing agent, a synthetic based scrubbing agent and a mineral based scrubbing agent.

10. The composition according to claim 9 wherein the vegetable based scrubbing agent is any one or combination of cornmeal, olive stone, walnut shells, ground fruit stones, ground corn meal, ground fruit shells.

11. The composition according to claim 9 wherein the synthetic based scrubbing agent is any one or combination of polyethylene and polypropylene.

12. The composition according to claim 9 wherein the mineral based scrubbing agent is any one or combination of ground shellfish, pumice, and silica.

13. The composition according to any one of claims 1 to 12 wherein said skin conditioner is any one or combination of a polyol, an anionic surfactant, a non-ionic surfactant, a cationic surfactant, an amphoteric surfactant, a cationic polymer, a quaternised gum, and a polyol.

14. The composition according to claim 13 wherein said polyol is any one or combination of glycerine and polyglycerin-6, propylene glycol, sorbitol, mannitol, erythritol, xylitol, arabitol, ribitol, dulcitol, lactitol, and maltitol.

15. The composition according to any one of claims 1 to 14 including an antioxidant present in a range from 0.01 to 1.0% w/w.

16. The composition according to claim 15 wherein said antioxidant is any one or combination of butylhydroxytoluene (BHT), butylhydroxyanizole, 1,2-dihydroxybenzene, p-coumarine acid, caffeic acid, sodium sulfite, sodium metasulfite, ferrulic acid, tyrosol, quercetin, chlorogenic acid, oleuropein hydroxytyrosol, ascorbic acid, phenolic acid, propyl gallate, $\alpha$-tocopherol, $\beta$-tocopherol, $\gamma$-tocopherol, $\delta$-tocopherol, and tetradibutyl-pentaerithrityl hydroxyhydrocinnamate.

17. A method of producing and dispensing a gritty foam, the method comprising;
providing an unpressurized container having a dispenser pump configured to mix air with a foamable gritty composition under low pressure conditions, the foamable gritty composition comprising

    a) constituents including

        a solvent present in a range from 0.5% w/w to 30.0% w/w, said solvent including any one or combination of D'limonene and sunflower oil methyl ester;
        a particulate scrubbing agent including mechanical scrubber particles of from 100 to 800 microns in size, present in a range from 1.0% w/w to 8% w/w;
        a surfactant present in a range from 0.5% w/w to 30.0% w/w;
        skin conditioner present in a range from 0.01% w/w to 5.00% w/w; and
        a non-Newtonian thickening agent present in a range from 1.5% w/w to 1.7% w/w;
        water; and

    b) wherein the non-Newtonian thickening agent is selected from: acrylates/C10-30 alkyl acrylate crosspolymer, carbomers, xanthan gum, guar gum, quaternised guar gum, alginate, bentonite, fumed silica or combination thereof,
    mixing air with said foamable gritty composition in said unpressurized foam dispenser under low pressure conditions; and
    dispensing said foamable gritty composition as a gritty foam from said unpressurized foam dispenser.

18. The method of claim 17 including
introducing a predetermined quantity of air under pressure to a first side of a microporous air sparging element located upstream from an outlet;
introducing, concurrently with the introduction of the air, a predetermined amount of said foamable gritty composition under pressure to a foamable gritty composition inlet of a mixing chamber located on a second side of the microporous air sparging element that is located upstream from said outlet, such that a ratio of air to foamable gritty composition has a greater proportion of air to foamable gritty composition;
forcing the air through the microporous sparging element into the mixing chamber to form a plurality of bubbles;
mixing the plurality of bubbles and the foamable gritty composition in the mixing chamber thereby creating a gritty foam; and
dispensing the gritty foam to a user in shots through said outlet from the mixing chamber.

**19.** A method as claimed in claim 18 wherein the air is introduced a predetermined quantity and the foamable gritty composition is introduced in a predetermined quantity.

**Patentansprüche**

**1.** Schäumbare körnige Zusammensetzung, umfassend:

a) Inhaltsstoffe einschließlich

einem Lösungsmittel, das in einem Bereich von 0,5 Gew.-% bis 30,0 Gew.-% enthalten ist, wobei das Lösungsmittel eine beliebige oder eine Kombination der Substanzen D-Limonen und Sonnenblumenölmethylester enthält;

einem teilchenhaltigen Scheuermittel, das mechanische Scheuerteilchen im Größenbereich zwischen 100 $\mu$m und 800 $\mu$m enthält, das in einem Anteilsbereich von 1,0 Gew.-% bis 8 Gew.-% vorhanden ist;

einem Tensid, das in einem Anteilsbereich von 0,5 Gew.-% bis 30,0 Gew.-% vorhanden ist; Hautkonditionierer, vorhanden in einem Anteilsbereich von 0,01 Gew.-% bis 5,00 Gew.-%; und

einem nicht-newtonschen Verdickungsmittel, das in einem Anteilsbereich von 1,5 Gew.-% bis 1,7 Gew.-% vorhanden ist;

Wasser; und

b) bei der das nicht-newtonsche Verdickungsmittel ausgewählt ist aus: Acrylate/C10-30-Alkylacrylat-Kreuzpolymer, Carbomeren, Xanthangummi, Guargummi, quaternisiertem Guargummi, Alginat, Bentonit, pyrogener Kieselsäure oder einer Kombination davon, derart, dass die schäumbare körnige Zusammensetzung als körniger Schaum aus einem Nicht-Aerosol-Schaumspender ausgegeben werden kann, der zur Ausgabe von Handreinigungsmit-teln fähig ist, welche die mechanischen Scheuerteilchen enthalten.

**2.** Zusammensetzung nach Anspruch 1, bei der das Tensid eine beliebige oder eine Kombination folgender Substanzen aufweist:

i) ein anionisches Tensid, das in einem Anteilsbereich von 1 Gew.-% bis 20 Gew.-% vorhanden ist, soweit es alleine vorliegt;

ii) ein amphoteres Tensid, das in einem Anteilsbereich von 0,5 Gew.-% bis 5,0 Gew.-% vorhanden ist, soweit es alleine vorliegt;

iii) ein nichtionisches Tensid, das in einem Anteilsbereich von 0,5 Gew.-% bis 20 Gew.-% vorhanden ist, soweit es alleine vorliegt; und

bei der die Gesamtmenge des Tensids oder einer beliebigen Kombination von vorhandenen Tensiden in einem Anteilsbereich von 0,5 Gew.-% bis 30,0 Gew.-% liegt.

**3.** Zusammensetzung nach Anspruch 2, bei der das anionische Tensid ausgewählt ist aus der Gruppe bestehend aus Laurylsulfaten, Laurylethersulfaten, Sulfosuccinaten, Carboxylaten (d.h. Natriumoleat), Carbonsäureestern (d.h. Natriumdilaurethcitrat), Alkylsulfat (d.h. Natriumlaurylethersulfat, Ammoniumalkylsulfat), Alkyl- und Alkylarylsulfonate (d.h. Natriumdodecylbenzolsulfonat), Sulfosuccinate (d.h. Dinatriumlaurylethersulfosuccinat), Isethionate (d.h. Natriumcocoylisethionat, Ammoniumcocoylisethionat), Taurate (d.h. Natriummethylcocoyltaurat, Natriummethyloleoyltaurat), Acylglutamate (d.h. Natriumlauroylglutamat, Natriumcocoylglutamat, Dinatriumcocoylglutamat), Sarcosinat (d. h. Cocoylsarcosinat), Alkylpolyglucoside (d. h. Decylglucosid, Natriumlaurylglucosecarboxylat, Caprylyl/Caprylglucosid).

**4.** Zusammensetzung nach Anspruch 2, bei der das amphotere Tensid eine beliebige oder eine Kombination der Substanzen Betaine, Acylethylendiamine, Aminosäurederivate, Imidazoline ist.

**5.** Zusammensetzung nach Anspruch 2, bei der das amphotere Tensid eine beliebige oder eine Kombination der Substanzen Acylamphoacetat, Acylamphodiacetat, Acylamphodipropionat, Natriumcocoglycinat, Natriumalkyliminodipropionat, Cocamidopropylbetain, Natriumcocoampho-acetat ist.

**6.** Zusammensetzung nach Anspruch 4, bei der das Betain aus der Gruppe ausgewählt ist, die aus Cocobetain und Cocamidopropyl-Betain besteht.

**7.** Zusammensetzung nach Anspruch 2, bei der das nichtionische Tensid eine beliebige oder eine Kombination der Substanzen Glucoside, ethoxylierten Fettalkohole, ethoxylierte Fettsäuren, Saccharoseester, Sorbitanester, Alkanolamide, Glycerinalkylester und Polyoxyethylenglykolalkylphenolether ist.

8. Zusammensetzung nach einem der Ansprüche 1 bis 7, bei der das Lösungsmittel weiterhin eine beliebige oder eine Kombination der Substanzen Glykolether, Ester, Alkohole, Terpene außer D-Limonen, aromatenfreier Terpentinersatz enthält und bei der die Gesamtmenge des vorhandenen Lösungsmittels in dem Anteilsbereich von 0,5 Gew.-% bis 30,0 Gew.-% liegt.

9. Zusammensetzung nach einem der Ansprüche 1 bis 8, bei der das teilchenhaltige Scheuermittel ein beliebiges oder eine Kombination folgender Scheuermittel ist: Scheuermittel auf pflanzlicher Basis, Scheuermittel auf synthetischer Basis oder Scheuermittel auf Mineralbasis.

10. Zusammensetzung nach Anspruch 9, bei der das Scheuermittel auf pflanzlicher Basis eine beliebige oder eine Kombination der Substanzen Maismehl, Olivenkern, Walnussschalen, gemahlenen Obstkerne, gemahlenes Maismehl, gemahlene Fruchtschalen ist.

11. Zusammensetzung nach Anspruch 9, bei der das Scheuermittel auf synthetischer Basis eine beliebige oder eine Kombination der Substanzen Polyethylen und Polypropylen ist.

12. Zusammensetzung nach Anspruch 9, bei der das Scheuermittel auf Mineralbasis eine beliebige oder eine Kombination der Substanzen gemahlene Schalentiere, Bimsstein und Siliciumdioxid ist.

13. Zusammensetzung nach einem der Ansprüche 1 bis 12, bei der der Hautkonditionierer eine beliebige oder eine Kombination der Substanzen Polyol, anionisches Tensid, nichtionisches Tensid, kationisches Tensid, amphoteres Tensid, kationisches Polymer, quaternisiertes Gummi, und ein Polyol ist.

14. Zusammensetzung nach Anspruch 13, bei der das Polyol eine beliebige oder eine Kombination der Substanzen Glycerin und Polyglycerin-6, Propylenglykol, Sorbitol, Mannitol, Erythritol, Xylitol, Arabitol, Ribitol, Dulcitol, Lactitol und Maltitol ist.

15. Zusammensetzung nach einem der Ansprüche 1 bis 14 einschließlich eines Antioxidationsmittels, das in einem Anteilsbereich von 0,01 bis 1,0 Gew.-% vorliegt.

16. Zusammensetzung nach Anspruch 15, bei der das Antioxidationsmittel eine beliebige oder eine Kombination der Substanzen Butylhydroxytoluol (BHT), Butylhydroxyanizol, 1,2-Dihydroxy-benzol, p-Cumarinsäure, Koffeinsäure, Natriumsulfit, Natriummetasulfit, Ferrulsäure, Tyrosol, Quercetin, Chlorogensäure, Oleuropein-Hydroxytyrosol, Ascorbinsäure, Phenolsäure, Propyl-gallat, $\alpha$-Tocopherol, $\beta$-Tocopherol, $\gamma$-Tocopherol, $\delta$-Tocopherol und Tetradibutyl-Pentaerithrityl-Hydroxyhydrocinnamat ist.

17. Verfahren zur Herstellung und Ausgabe eines körnigen Schaums, wobei das Verfahren folgende Schritte umfasst:

- Bereitstellen eines drucklosen Behälters mit einer Spenderpumpe, die konfiguriert ist, Luft mit einer schäumbaren körnigen Zusammensetzung unter Unterdruckbedingungen zu mischen, wobei die schäumbare körnige Zusammensetzung Folgendes umfasst:

a) Inhaltsstoffe einschließlich
einem Lösungsmittel, das in einem Bereich von 0,5 Gew.-% bis 30,0 Gew.-% enthalten ist, wobei das Lösungsmittel eine beliebige oder eine Kombination der Substanzen D-Limonen und Sonnenblumenölmethylester enthält;
einem teilchenhaltigen Scheuermittel, das mechanische Scheuerteilchen im Größenbereich zwischen 100 $\mu$m und 800 $\mu$m enthält, das in einem Anteilsbereich von 1,0 Gew.-% bis 8 Gew.-% vorhanden ist;
einem Tensid, das in einem Anteilsbereich von 0,5 Gew.-% bis 30,0 Gew.-% vorhanden ist; Hautkonditionierer, vorhanden in einem Anteilsbereich von 0,01 Gew.-% bis 5,00 Gew.-%; und
einem nicht-newtonschen Verdickungsmittel, das in einem Anteilsbereich von 1,5 Gew.-% bis 1,7 Gew.-% vorhanden ist;
Wasser; und
b) bei der das nicht-newtonsche Verdickungsmittel ausgewählt ist aus: Acrylate/C10-30-Alkylacrylat-Kreuzpolymer, Carbomeren, Xanthangummi, Guargummi, quaternisiertem Guargummi, Alginat, Bentonit, pyrogener Kieselsäure oder einer Kombination davon, derart, dass die schäumbare körnige Zusammensetzung als körniger Schaum aus einem Nicht-Aerosol-Schaumspender ausgegeben werden kann, der zur Ausgabe von Handreinigungsmitteln fähig ist, welche die mechanischen Scheuerteilchen enthalten, Mischen von Luft mit der schäumbaren körnigen Zusammensetzung in dem drucklosen Schaumspender unter Unterdruckbedingungen; und

Ausgabe der schäumbaren körnigen Zusammensetzung als körniger Schaum aus dem drucklosen Schaumspender.

**18.** Verfahren nach Anspruch 17 einschließlich folgender Schritte:

- Einleiten einer vorbestimmten Menge Druckluft auf einer ersten Seite eines mikroporösen Luftdurchblaselements, das sich stromaufwärts von einem Auslass befindet;
- gleichzeitig mit der Lufteinleitung: Einleiten einer vorbestimmten Menge der schäumbaren körnigen Zusammensetzung unter Druck in einen Einlass für schäumbare körnige Zusammensetzung einer Mischkammer, die auf einer zweiten Seite des mikroporösen Luftdurchblaselements angeordnet ist, das stromaufwärts von dem Auslass angeordnet ist, derart, dass ein Verhältnis von Luft zu schäumbarer körniger Zusammensetzung einen größeren Anteil an Luft zu schäumbarer körniger Zusammensetzung aufweist;
- Pressen der Luft durch das mikroporöse Durchblaselement hindurch in die Mischkammer unter Bildung einer Vielzahl von Blasen;
- Mischen der Vielzahl von Blasen und der schäumbaren körnigen Zusammensetzung in der Mischkammer, wodurch ein körniger Schaum entsteht; und
- stoßweise Ausgabe des körnigen Schaums an einen Benutzer über den genannten Auslass aus der Mischkammer.

**19.** Verfahren nach Anspruch 18, bei dem die Lufteinleitung in einer vorbestimmten Menge erfolgt und die Einleitung der schäumbaren körnigen Zusammensetzung in einer vorbestimmten Menge erfolgt.

**Revendications**

**1.** Composition granuleuse pouvant être mise sous forme de mousse, comprenant:

a) des constituants incluant
un solvant présent dans une plage de 0,5% p/p à 30,0% p/p, ledit solvant incluant l'un quelconque ou une combinaison de D'limonène et d'ester méthylique d'huile de tournesol;
un agent de nettoyage particulaire incluant des particules de nettoyant mécanique d'une taille de 100 à 800 microns, présent dans une plage de 1,0% p/p à 8% p/p;
un tensioactif présent dans une plage de 0,5% p/p à 30,0% p/p;
un revitalisant pour la peau présent dans une plage de 0,01% p/p à 5,00% p/p; et
un agent épaississant non newtonien présent dans une plage de 1,5% p/p à 1,7% p/p;
de l'eau; et
b) où l'agent épaississant non newtonien est choisi parmi: un polymère réticulé acrylates/acrylate d'alkyle en C10-30, les carbomères, la gomme de xanthane, la gomme de guar, la gomme de guar quaternisée, l'alginate, la bentonite, la silice fumée ou une combinaison de ceux-ci,
de telle sorte que ladite composition granuleuse pouvant être mise sous forme de mousse peut être distribuée sous forme de mousse granuleuse à partir d'un distributeur de mousse sans aérosol capable de distribuer des nettoyants pour les mains avec lesdites particules de nettoyant mécanique présentes à l'intérieur.

**2.** Composition selon la revendication 1, où le tensioactif inclut l'un quelconque ou une combinaison de

i) un tensioactif anionique présent dans une plage de 1% p/p à 20% p/p s'il est présent seul;
ii) un tensioactif amphotère présent dans une plage de 0,5% p/p à 5,0% p/p s'il est présent seul;
iii) un tensioactif non ionique présent dans une plage de 0,5% p/p à 20% p/p s'il est présent seul; et

où une quantité totale de tensioactif ou de toute combinaison de tensioactifs présents est dans une plage de 0,5% p/p à 30,0% p/p.

**3.** Composition selon la revendication 2 où le tensioactif anionique est choisi dans le groupe consistant en les laurylsulfates, les lauryléthersulfates, les sulfosuccinates, les carboxylates (c'est-à-dire oléate de sodium), les esters d'acide carboxylique (c'est-à-dire dilaureth citrate de sodium), les alkylsulfates (c'est-à-dire lauryléthersulfate de sodium, alkylsulfate d'ammonium, alkyl et alkyl-arylsulfonates (c'est-à-dire dodécylbenzènesulfonate de sodium), les sulfosuccinates (c'est-à-dire lauryléthersulfosuccinate disodique), les iséthionates (c'est-à-dire cocoyl iséthionate de sodium, cocoyl iséthionate d'ammonium), les taurates (c'est-à-dire méthyl cocoyl taurate de sodium, méthyl oléoyl taurate de sodium), les acyl glutamates (c'est-à-dire lauroyl glutamate de sodium, cocoyl glutamate de sodium, cocoyl glutamate disodique), le sarcosinate (c'est-à-dire cocoyl sarcosinate), les alkylpolyglucosides (c'est-à-dire décylglucoside, lauryl glucose carboxylate de sodium, caprylyl/capryl glucoside).

4. Composition selon la revendication 2, où le tensioactif amphotère est l'un quelconque ou une combinaison de bétaïnes, acyléthylène diamines, dérivés d'acides aminés, imidazolines.

5. Composition selon la revendication 2, où le tensioactif amphotère est l'un quelconque ou une combinaison d'acyl-lamphoacétate, d'acylamphodiacétate, d'acylamphodipropionate, de cocoglycinate de sodium, d'alkyliminodipropionate de sodium, de cocamidopropyl bétaïne, de cocoamphoacétate de sodium.

6. Composition selon la revendication 4, où ladite bétaïne est choisie dans le groupe consistant en la coco bétaïne et la cocamidopropyl bétaïne.

7. Composition selon la revendication 2, où le tensioactif non ionique est l'un quelconque ou une combinaison de glucosides, d'alcools gras éthoxylés, d'acides gras éthoxylés, d'esters de saccharose, d'esters de sorbitane, d'alcanolamides, d'alkylesters de glycérol et d'éthers d'alkylphénol et de polyoxyéthylèneglycol.

8. Composition selon l'une quelconque des revendications 1 à 7, où le solvant inclut en outre l'un quelconque ou une combinaison d'éthers de glycol, d'esters, d'alcools, de terpènes autres que le D'Limonène, de white-spirit sans aromatique, et où une quantité totale de solvant présent est dans ladite plage de 0,5% p/p à 30,0% p/p.

9. Composition selon l'une quelconque des revendications 1 à 8, où l'agent de nettoyage particulaire est l'un quelconque ou une combinaison d'un agent de nettoyage à base végétale, d'un agent de nettoyage à base synthétique et d'un agent de nettoyage à base minérale.

10. Composition selon la revendication 9, où l'agent de nettoyage à base végétale est l'un quelconque ou une combinaison de farine de maïs, de noyau d'olive, de coquilles de noix, de noyaux de fruits moulus, de farine de maïs moulu, de coquilles de fruits moulues.

11. Composition selon la revendication 9, où l'agent de nettoyage à base synthétique est l'un quelconque ou une combinaison de polyéthylène et de polypropylène.

12. Composition selon la revendication 9, où l'agent de nettoyage à base minérale est l'un quelconque ou une combinaison de mollusques moulus, de pierre ponce et de silice.

13. Composition selon l'une quelconque des revendications 1 à 12 où ledit revitalisant pour la peau est l'un quelconque ou une combinaison d'un polyol, d'un tensioactif anionique, d'un tensioactif non ionique, d'un tensioactif cationique, d'un tensioactif amphotère, d'un polymère cationique, d'une gomme quaternisée et d'un polyol.

14. Composition selon la revendication 13, où ledit polyol est l'un quelconque ou une combinaison de glycérine et de polyglycérine-6, de propylèneglycol, de sorbitol, de mannitol, d'érythritol, de xylitol, d'arabitol, de ribitol, de dulcitol, de lactitol et de maltitol.

15. Composition selon l'une quelconque des revendications 1 à 14 incluant un antioxydant présent dans une plage de 0,01 à 1,0% p/p.

16. Composition selon la revendication 15, où ledit antioxydant est l'un quelconque ou une combinaison de butylhydroxytoluène (BHT), butylhydroxyanizole, 1,2-dihydroxybenzène, acide p-coumarique, acide caféique, sulfite de sodium, métasulfite de sodium, acide ferrulique, tyrosol, quercétine, acide chlorogénique, oleuropéine hydroxytyrosol, acide ascorbique, acide phénolique, gallate de propyle, $\alpha$-tocophérol, $\beta$-tocophérol, $\gamma$-tocophérol, $\delta$-tocophérol et tétradibutyl-pentaérithrityl hydroxyhydrocinnamate.

**17.** Procédé de production et de distribution d'une mousse granuleuse, le procédé comprenant;
la fourniture d'un récipient non sous pression ayant une pompe de distribution configurée pour mélanger de l'air avec une composition granuleuse pouvant être mise sous forme de mousse dans des conditions de basse pression, la composition granuleuse pouvant être mise sous forme de mousse comprenant

a) des constituants incluant
un solvant présent dans une plage de 0,5% p/p à 30,0% p/p, ledit solvant incluant l'un quelconque ou une combinaison de D'limonène et d'ester méthylique d'huile de tournesol;
un agent de nettoyage particulaire incluant des particules de nettoyant mécanique d'une taille de 100 à 800 microns, présentes dans une plage de 1,0% p/p à 8% p/p;
un tensioactif présent dans une plage de 0,5% p/p à 30,0% p/p;
un revitalisant pour la peau présent dans une plage de 0,01% p/p à 5,00% p/p; et
un agent épaississant non newtonien présent dans une plage de 1,5% p/p à 1,7% p/p;
de l'eau; et
b) où l'agent épaississant non newtonien est choisi parmi:
un polymère réticulé acrylates/acrylate d'alkyle en C10-30, les carbomères, la gomme de xanthane, la gomme de guar, la gomme de guar quaternisée, l'alginate, la bentonite, la silice fumée ou une combinaison de ceux-ci,
le mélange d'air avec ladite composition granuleuse pouvant être mise sous forme de mousse dans ledit distributeur de mousse non sous pression dans des conditions de basse pression; et
la distribution de ladite composition granuleuse pouvant être mise sous forme de mousse sous forme d'une mousse granuleuse depuis ledit distributeur de mousse non sous pression.

**18.** Procédé selon la revendication 17, incluant
l'introduction d'une quantité prédéterminée d'air sous pression d'un premier côté d'un élément de barbotage d'air microporeux situé en amont d'une sortie;
l'introduction, en même temps que l'introduction de l'air, d'une quantité prédéterminée de ladite composition granuleuse pouvant être mise sous forme de mousse sous pression dans une entrée de composition granuleuse pouvant être mise sous forme de mousse d'une chambre de mélange située sur un second côté de l'élément de barbotage d'air microporeux qui est situé en amont de ladite sortie, de sorte qu'un rapport d'air à composition granuleuse pouvant être mise sous forme de mousse a une plus grande proportion d'air à composition granuleuse pouvant être mise sous forme de mousse;
le fait de forcer l'air à travers l'élément de barbotage microporeux dans la chambre de mélange pour former une pluralité de bulles;
le mélange de la pluralité de bulles et de la composition granuleuse pouvant être mise sous forme de mousse dans la chambre de mélange, pour créer ainsi une mousse granuleuse; et
la distribution de la mousse granuleuse à un utilisateur par coups à travers ladite sortie de la chambre de mélange.

**19.** Procédé selon la revendication 18, où l'air est introduit en une quantité prédéterminée et la composition granuleuse pouvant être mise sous forme de mousse est introduite en une quantité prédéterminée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8002151 B

- US 8281958 B